# EUROPEAN PATENT APPLICATION

(11) **EP 4 382 476 A1**
(43) Date of publication of application: **12.06.2024**
(21) Application number: 22290065.6
(22) Date of filing: 06.12.2022
(51) Int. Cl.: C01B 3/16, C01B 3/38

(54) **SYSTEM AND PROCESS FOR PRODUCING SYNTHETIC FUELS**

(71) Applicant: Technip Energies France, 92741 Nanterre Cedex (FR)
(72) Inventor: Riegman, Jan-Jaap, 92741 Nanterre Cedex (FR); Hamdi, Mehdi, 92741 Nanterre Cedex (FR)
(74) Representative: McWilliams, David John

(57) **Abstract**

System for producing synthetic fuels comprising:
- A reforming reactor (7) configured to react a light hydrocarbon feed (24) together with an oxidant gas stream (20) thereby producing a reformer effluent comprising a synthesis gas stream, and
- A reverse water gas shift reactor (6) configured to receive a CO2 rich stream, a H2 rich stream and the reformer effluent and to produce a synthesis gas with a reduced H2/CO ratio,
- A synthesis unit (17) configured to receive the synthesis gas with a reduced H2/CO ratio and to produce a feed comprising synthetic fuels (18),
With the reverse water gas shift reactor (17) comprising a vessel comprising:
• at least one catalyst zone, preferably a catalyst tube, configured to receive the H2 rich stream and the CO2 rich stream and to produce a shift effluent enriched in CO and H2O,
• a heat transfer zone configured to receive the reformer effluent and to transfer the heat of the reformer effluent to the catalyst zone,
and
means configured to mix the shift effluent with the reformer effluent and to produce the synthesis gas with the reduced H2/CO ratio.

## Description

The invention is related to a system and a process for producing synthetic fuels.

Synthetic fuel or synfuel is a liquid fuel, or sometimes gaseous fuel, obtained from syngas (synthesis gas), a mixture of carbon monoxide and hydrogen, in which the syngas was derived from gasification of solid feedstocks such as coal or biomass or by reforming of natural gas.

Currently, it is very important to develop processes that allow utilization of alternative energy sources to provide fuel that can immediately substitute the currently usedhydrocarbon fuels. Such fuel which is compatible with combustion engines as they are today will render the need for major, time consuming, technical developments and infrastructural changes unnecessary. Of particular interest is the production of Sustainable Aviation Fuel (SAF) as this is a hard to decarbonize industry.

One process on industrial scale to produce synthetic liquid hydrocarbon fuel is based on classic Fischer-Tropsch chemistry and converts syngas to a variety of hydrocarbons via the Fischer-Tropsch synthesis.

Synthesis gas is traditionally produced from reforming of fossil hydrocarbons such as natural gas, LPG (Liquefied Petroleum Gas) or naphtha. The hydrocarbon feedstock is pretreated to remove impurities, before steam is added to the feedstock. The steam/hydrocarbon mixture is heated further up to the inlet temperature of the reforming section. In the reforming section the hydrocarbon and steam mixture is reacting to form a syngas mixture including hydrogen, carbonmonoxide, carbon dioxide, non-reacted steam and a small amount of unconverted methane as well as in case present some inerts such as nitrogen. The reaction is endothermic, so the conversion of hydrocarbons promoted by high outlet temperatures preferably at least 800C and higher. The process gas is cooled down and condensed water is removed from the process gas and the CO2 is captured to form a CO2 depleted process gas. Depending on the desired product quality and composition further separation steps are included.

More details of various processes for producing synthesis gas with low H2/CO- ratio can be found in"Industrial scale experience on steam reforming of CO2- rich gas", P.M. Mortensen & I. Dybkjasr, Applied Catalysis A: General, 495 (2015), 141-151.

The terms "reforming" and "methane reforming" are meant to denote a reforming reaction according to one or more of the following reactions:

CH4+ H20 < CO + 3H2 (i)

CH4+ 2H20 < C02+ 4H2 (ii)

CH4+ C02 <2CO + 2H2 (iii)

Reactions (i) and (ii) are steam methane reforming reactions, whilst reaction (iii) is the dry methane reforming reaction.

For higher hydrocarbons, viz. CnHm, where n≥2, m ≥ 4, equation (i) is general- ized as: CnHm+ n H2O -> nCO + (n + m/2)H2(iv), where n≥2, m > 4

Typically, reforming is accompanied by the water gas shift reaction (v):

CO + H20 < C02+ H2 (V)

The term "steam methane reforming" is meant to cover the reactions (i) and (ii) running from the left towards the right side of the arrow (i.e. conversion of methane), whilst the term "methanation" is meant to cover the reactions (i) and/or (ii) running from the right towards the left side of the arrow (i.e. formation of methane). Thus, the term "steam methane reforming/methanation reactions" is meant to denote the reactions (i) and (ii) running towards equilibrium. The term "reverse water gas shift" is meant to denote the reaction (v) running from the right towards the left side of the arrow. In most cases, all of these reactions are at, or close to, equilibrium at the outlet from the catalyst bed or catalyst zone of the reactor concerned.

Processes based on Autothermal Reforming (ATR) is an alternative route to production of synthesis gas, especially when a low ratio of hydrogen to carbon monoxide is required. The main elements of an ATR reactor are a burner, a combustion chamber, and a catalyst bed contained within a refractory lined pressure shell. In an ATR reactor, partial combustion of the hydrocarbon feed by sub-stoichiometric amounts of oxygen is followed by steam reforming of the partially combusted hydrocarbon feed stream in a fixed bed of reforming catalyst. Steam reforming also takes place to some extent in the combustion chamber due to the high temperature. The steam reforming reaction is accompanied by the water gas shift reaction. Typically, the gas is at or close to equilibrium at the outlet of the reactor with respect to steam reforming and water gas shift reactions. The temperature of the exit gas is typically in the range between 850 and 1100°C. More details of ATR and a full description can be found in the art such as "Studies in Surface Science and Catalysis, Vol. 152," Synthesis gas production for FT synthesis"; Chapter 4, p.258-352, 2004".

ATR uses oxygen and steam, and optionally also carbon dioxide, in a reaction with a hydrocarbon feed stream to form synthesis gas. The ratio of hydrogen to carbon monoxide in the exit gas depends upon the selected operation conditions including the amount of steam and carbon dioxide added to the hydrocarbon feed stream and/or the ATR reactor. The reforming section can comprise of a prereformer, steam methane reformer, an autothermal reformer or any combination thereof. For all reforming options, increasing the amount of carbon dioxide in the feed will decrease the hydrogen to carbon monoxide ratio in the product gas, but will also increase the size of the reactor due to the higher flow as well as increase the risk for the formation of carbon (and therefore typically requires an increase steam to carbon flowrate and consequently a reduced overall energy efficiency).

Depending on the downstream synthesis the different H2/CO ratios, for example Fischer-Tropsch synthesis depending on product and catalyst type the optimum H2/CO ratio can be as low as 0.6 for iron based catalyst or 1.8-2.1 for a cobalt based catalyst.

Several processes for producing synthetic fuels comprising the Fischer-Tropsch synthesis are known but these processes are often characterized by a high hydrogen utilization rate and a high power consumption (especially related to the hydrogen production by electrolysis).

The aim of the invention is to provide an improved process and an improved system for producing synthetic fuels.

A solution of the present invention is a system for producing synthetic fuels comprising:
- A reforming reactor 7 configured to react a light hydrocarbon feed 24 together with an oxidant gas stream 20 thereby producing a reformer effluent comprising a synthesis gas stream, and
- A reverse water gas shift reactor 6 (rWGS) configured to receive a CO2 rich stream, a H2 rich stream and the reformer effluent and to produce a synthesis gas with a reduced H2/CO ratio,
- A synthesis unit 17 configured to receive the synthesis gas with a reduced H2/CO ratio and to produce a feed comprising synthetic fuels 18,

With the reverse water gas shift reactor 17 comprising a vessel comprising:
- at least one catalyst zone, preferably a catalyst tube, configured to receive the H2 rich stream and the CO2 rich stream and to produce a shift effluent enriched in CO and H2O,
- a heat transfer zone configured to receive the reformer effluent and to transfer the heat of the reformer effluent to the catalyst zone,
and
means configured to mix the shift effluent with the reformer effluent and to produce the synthesis gas with the reduced H2/CO ratio.

The term "reforming reactor" is meant to denote a synthesis gas generation reactor, such as i.e a steam methane reforming reactor, prereforming reactor, dry reforming reactor, an autothermal reforming reactor or a combination thereof.

The term "synthesis gas" is meant to cover a gas comprising at least hydrogen and carbon monoxide, while it also may comprise carbon dioxide, methane and steam and possibly small amounts of other gasses, such as argon, nitrogen, etc.

In an embodiment, the reforming reactor is an ATR reactor. The ATR reactor comprises a burner, a combustion chamber, and a bed of the first catalyst housed within a refractory lined pressure shell. In another embodiment, the reforming reactor is a steam methane reforming reactor. The steam methane reforming reactor comprises a number of tubes housing the first catalyst within a furnace with burners.

The product gas stream is a synthesis gas with the reduced H2/CO ratio. The term "reduced" is used in comparison with the synthesis gas comprised in the reformer effluent. Preferably, the product gas stream is a synthesis gas comprising an H2/CO ratio below 3.0, preferably below 2.0, more preferably below 1.8, even more preferably below 1.6, and most preferably below 1.4.

The temperature of the reformer effluent is comprised between 500 and 1100°C, more preferably between 800 and 1100°C. So the reformer effluent is providing the heat for the reverse water gas shift reaction as well heating up the gas to the required exit temperature (preferably more than 750°C). The CO2 rich and hydrogen rich gasses are mixed upstream and preheated to the required inlet temperature before entering the catalyst tubes of the reverse water gas shift reactor (preferably more than 400°C, more preferably between 600 and 700°C). The preheating can be achieved by either and external heat exchanger, electrical heater or fired heater or inside the reverse water gas shift reactor by applying a heat transfer promoting non catalytic insert above the catalyst bed in the catalyst tube. The preheated CO2-H2 mixture fed through the catalyst bed inside the catalyst tubes.

The catalyst shall be high temperature resistant and active for at least the reverse water gas shift reaction and typically also for methanation reaction, although this is preferably suppressed to a minimum. The catalyst is typically Nickel based due to thermal stability as well as availability. Alternatively noble metal catalysts or Iron and/or Copper based catalyst can be applied.

Depending on the embodiment, the system according to the present invention can comprise one or more of the following features:
- the synthesis unit 17 is any of the following units or a combination of any of these: a methanol synthesis unit, a methanol synthesis unit combined with a methanol to gasoline or methanol to jet fuel unit, a syngas to olefines synthesis unit, a syngas to ethyleneoxide unit and a Fischer Tropsch FT unit.
- the system comprises at least a separation unit 19 configured to receive the feed comprising synthetic fuels 18 coming from the synthesis unit (17) and to produce synthetic fuels 21, water 26 and a feed comprising light hydrocarbons (22); and preferably the reforming reactor 7 is configured to receive the light hydrocarbons coming from the separation unit 19;
- The system comprises a first CO2 capture unit 23 configured to receive the feed comprising light hydrocarbons and to produce CO2 stream 25 and light hydrocarbons 24; and preferably the reforming reactor 7 is configured to receive the light hydrocarbons coming from the CO2 capture unit 23, and/or Preferably the reverse water gas shift reactor 6 is configured to receive the CO2 stream 25 coming from the CO2 capture unit 23.
- The system comprises an electrolyzer unit 3 configured to receive water 1 and to produce a H2 stream 4 and a O2 stream 20, the reverse water gas shift reactor 6 is configured to receive the H2 stream 4 coming from the electrolyzer unit 3, and preferably the reforming reactor 7 is configured to receive the O2 stream coming from the electrolyzer unit 3 as oxidant gas stream 20.
- the end of the catalyst zone opens in the heat transfer zone and means to mix configured to mix the shift effluent with the reformer effluent are located in the heat transfer zone of the vessel; this first configuration will then be called "option A";
- the end of the catalyst zone opens in a zone comprised in the vessel and fluidically isolated from the heat transfer zone and means to mix the shift effluent with the reformer effluent are located at the reverse water gas shift reactor outlet; This second configuration will then be called "option B";
- The system comprises a second CO2 capture unit 12 configured to receive the synthesis gas with the reduced H2/CO ratio and to produce CO2 stream 13 and a synthesis gas 16 depleted in CO2 and with the reduced H2/CO ratio, and preferably the catalyst zone is configured to receive a CO2 stream coming at least in part from the CO2 capture unit 12;
- the system comprises an external hydrogen source and the catalyst zone of the reverse gas shift reactor received hydrogen coming in part from the external hydrogen source;
- the top of the catalyst tube is filled with a catalytically inactive heat transfer enhancing component;
- the system comprises means to mix the hydrocarbon feed with an oxidant stream upstream the reforming reactor. By "means to mix" we mean for example a mixing tee;
- the first and/or the CO2 capture unit(s) is/are selected from an amine adsorption unit, a carbonate solution adsorption unit, a cryogenic unit, a membrane unit, electrochemical compression, temperature swing adsorption or a pressure swing adsorption unit;
- the reforming reactor comprises one or a combination of the following reforming units: prereformer, steam methane reformer, dry methane reformer, autothermal reformer or partial oxidation.

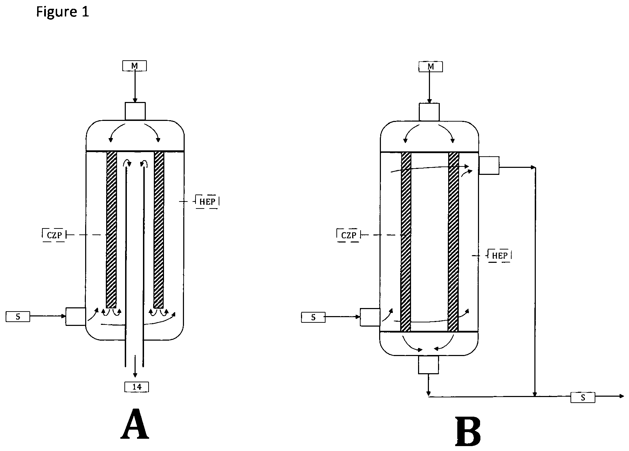

The figure 1 schematically indicates the two configurations (option A and option B) for the reverse water gas shift reactor. The preheated mixture of the CO2 rich stream and the H2 rich stream (M) enters the catalyst zone at minimum 400°C, preferably more than 600°C or more preferably more than 650°C to minimize methane formation as well as the risk for carbon formation. In case the top part of the catalyst tubes is utilized as a heat transfer promoted zone without catalytically active material (HZP), the inlet temperature can be lower, minimum ambient temperature, and the heat transfer promoted zone is sized such that the catalyst bed inlet temperature is minimum 400°C, preferably more than 600°C or more preferably more than 650°C. The catalyst zone (CZP) comprised in the catalyst tubes reacts CO2 with H2 to form CO and H2O following the reverse water gas shift reaction. As a side product some methane is formed through the methanation reaction. The outlet temperature of the catalyst bed (shift effluent) shall be at least 500°C and more preferably mor than 750°C and or preferably more than 850°C or typically up to 1000°C to maximize CO2 conversion as well as minimizing the methane formation. With option A, the end of the catalyst tube opens in the heat transfer zone and means to mix the shift effluent richer in CO and H2O with the reformer effluent are located in the heat transfer zone. By "means to mix shift effluent with the reformer effluent", we mean a mixing volume inside the reverse water gas shift reactor below the catalyst tube outlet and at the location of the reformer effluent inlet. Thus, the synthesis gas with a reduced H2/CO ratio is formed in the heat transfer zone (HTZ). The temperature of the synthesis gas with a reduced H2/CO ratio is comprised between 400 and 900°C at the outlet of the heat transfer zone. And it is collected in a collector pipe to exit the reactor (S). The collector can be either direct at the outlet of the heat transfer zone (I.e. at the top below the catalyst tube tubesheet) or located inside the reverse water gas shift reactor between the catalyst tubes and at the opposite of the catalyst tubes outlet. The later is a preferred solution in case of revamps scenarios as it there is less mechanical stresses and easier routing to connect to existing equipment. In option A, the reverse water gas shift reactor has thus two inlet stream and one outlet stream. The major benefit of this concept in relation to reverse water gas shift is the reduced potential for carburization by diluting the CO rich shift effluent with the less carbon rich reformer effluent and thus reducing the partial pressure of CO compared to the shift effluent.

Alternatively, option B can be applied. In this concept the preheated mixture CO2-H2 (M) reacts in the catalyst zone (CZP) of the catalyst tubes under equal conditions as for concept one, but the end of the catalyst tube opens in a zone comprised in the vessel and fluidically isolated from the heat transfer zone and means to mix shift effluent with the reformer effluent (to provide a synthesis gas with a reduced H2/CO ratio (S))are located at the reverse water gas shift reactor outlet and not in the reverse water gas shift reactor. Thus the shift effluent exits the reactor in a separate stream. The advantage of this concept is that a slightly higher temperature compared to option A. In fact, the heating gas is directly the reformer effluent before mixing with the shift effluent and thus 10-50°C higher reverse water gas shift catalyst outlet temperature compared to option A is feasible. Additional produced synthesis gas with a reduced H2/CO ratio can be further processed separately. I.e. the shift effluent can be decoupled from the reforming section. Alternatively the two products can be combined. As the reverse water gas shift reactor has a high potential for carburization the outlet system should be designed in accordance with this. Preferably, the reverse water gas shift catalyst tubes are coated to protect against carburization/ metal dusting. Alternatively, materials that have a low level of susceptiveness to carburization can be selected.

In one particular scenario, where the reverse water gas shift reactor inlet temperature is relatively low i.e below 400°C or preferably below 600°C and higher inlet temperatures of the catalyst bed is preferred, the top part of the tubes could be filled with a heat transfer enhancing catalytically non active inert component to first increase the CO2-H2 mixture temperature before entering the catalyst bed. Such inert layer could be for example ceramic balls or a metal insert promoting turbulence. This is particularly beneficial if the inlet temperature of the reverse water gas shift reactor is lower than 400°C, especially lower than 300°C or even down to as low as ambient temperature.

Another object of the present invention is a process for producing synthetic fuels, said process implementing a system according to the present invention and comprises:
a) Reforming step (7) of a hydrocarbon feed (24) with an oxidant gas stream (20) to produce a reformer effluent comprising a synthesis gas stream,
b) Heating step of the catalyst zone of the of the reverse water gas shift reactor by heat exchange with the reformer effluent,
c) Reverse water gas shift reaction step (6) of a CO2 rich stream and a H2 rich stream (5) in the catalyst zone of the reverse water gas shift reactor to produce a shift effluent enriched in CO and H2O,
d) Mixing step of the shift effluent with the reformer effluent to produce a synthesis gas with the reduced H2/CO ratio (9),
e) Synthesis step (17) of a feed comprising synthetic fuels (18) from the synthesis gas with a reduced H2/CO ratio (9).

Depending on the embodiment, the process according to the present invention can comprise one or more of the following features:
- the synthesis step is a synthesis step according to the Fischer Tropsch process;
- the process comprises a separation step of the feed comprising synthetic fuels 18 to produce synthetic fuels 21, water 26 and a feed 22 comprising light hydrocarbons; and preferably a recycling step of light hydrocarbons 24 in the reforming reactor 7
- the process comprises a first step of capture of CO2 comprised in the feed 22 comprising light hydrocarbons to produce CO2 stream 25 and light hydrocarbons 24, preferably a recycling step of light hydrocarbons 24 in the reforming reactor 7, and preferably a recycling step of the CO2 stream 25 in the reverse water gas shift reactor 6;
- H2 rich stream 5 comes from at least in part from an external hydrogen source;
- The process comprises an electrolysis step of water 1 to produce H2 stream 4 and a O2 stream 20; the O2 stream produced at the electrolysis step is preferably used as oxidant gas stream 20 in the reforming step 7; and the H2 stream produced at the electrolysis step is introduced in the reverse water gas shift reactor 6;
- The process comprises a cooling step of the synthesis gas 9 coming from step d);
- The process comprises a second step of capture of CO2 comprised in the synthesis gas with the reduced H2/CO ratio 9 to produce CO2 stream 13 and a synthesis gas 16 depleted in CO2 and with the reduced H2/CO ratio, and a recycling step of the CO2 stream 13 in the reverse water gas shift reactor 6. An alternative option of the process does not include the second CO2 capture step, when the CO2 concentration in the reduced H2/CO ratio syngas is already below the allowable limits.
- preferably a cooling step at a temperature between 900 and 300°C and is typically cooled down to 30-50°C to knock out water and to the operating temperature of the next processing step, for example e) or the separation, The cooling down is achieve by heat exchanger for example feed preheating, steam generation and superheating and BFW preheating. The heat recovery is typically maximized in order to minimize the external energy demand for the syngas generation;
- The process comprises a mixture step of the light hydrocarbons 24 with an oxidant stream 20 before the reforming step 7;
- at step c) the CO2 rich stream and the H2 rich stream are introduced in the catalyst zone at a temperature higher than 400°C, preferably higher than 600°C, in case of lower temperature a heat transfer non catalytically active layer is included on top of the catalyst to ensure the desired catalyst operating temperature;
- the reformer effluent is at a temperature comprised between 700 and1100°C.

The invention describes the integration of a reverse water gas shift (rWGS) reaction step with downstream synthesis unit producing a feed comprising synthetic fuels, in particular a Fischer Tropsch synthesis unit, to close the carbon loop and minimize the import hydrogen required.

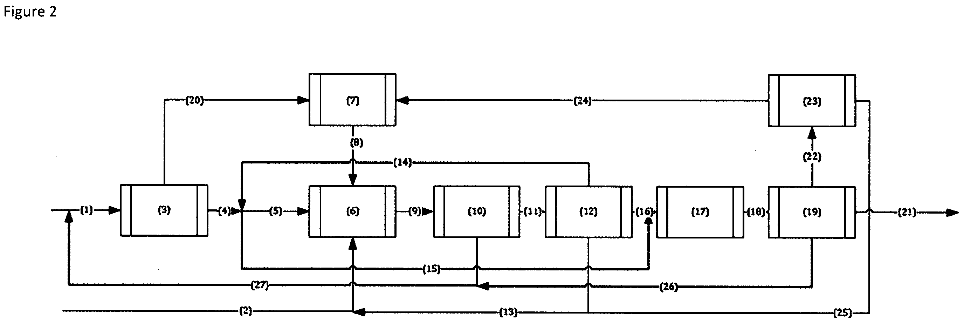

The figure 2 illustrate the process according to the present invention including optional features.

The process is an efficient path for utilizing CO2 while minimizing the hydrogen input (and thus power input) required to produce products.

The process is utilizing carbon dioxide and hydrogen to first produce a syngas and subsequently chemicals, in particular synthetic fuels for example Sustainable Aviation Fuels (SAF). A CO2 rich stream (2) is mixed with a H2 rich stream (4) and fed to a reverse water gas shift reactor (6).

The CO2 rich stream can be from any source with a high CO2 concentration, preferably >75% CO2, more in particular >90% CO2 and more in particular > 95% CO2. Lower CO2 concentration are feasible if the major other component in the stream is hydrogen, in this scenario less hydrogen should be mixed with the CO2 rich stream. The source is preferably a CO2 source from biogenic nature for example from a fermentation process or Direct Air Capture (DAC), although point sources from industry can also be treated without impact.

The imported hydrogen rich stream 4 for the process is typically generated in a electrolyzer unit 3, where water 1 is electrically dissociated into H2 and O2. Alternatively the hydrogen can also be generated in other processes, such as steam reforming, pyrolysis, partial oxidation or by separation from another hydrogen containing stream.

The reverse water gas shift reactor unit 6 convert CO2 and H2 to CO and a water byproduct over a catalyst. Before entering the reverse water gas shift reactor 6, the combined CO2 and H2 feed 5 is preferably preheated, additionally if beneficial also some steam can be blended with the feed. As the reaction is endothermic, the conversion of CO2 is higher at elevated temperatures and the catalyst bed outlet temperature is preferably 600-1000°C, more preferably above 750°C and more preferably above 800°C. The heat for the reverse water gas shift reactor is provided by hot reformer effluent 8 from the reforming section 7.

The outlet temperature of the combined gas 9, the synthesis gas with a reduced H2/CO ratio, from the reverse water gas shift reactor 6 is typically between 600-800°C and is fed to the cooling, condensation and separation section 10. The hot gas from the reverse water gas shift reactor (6) is cooled down and used for example steam generation, steam superheating, BFW ( = boiler feed water) preheating, Demin Water preparation and preheating as well as feed preheating. The water condensed in the cooled raw syngas stream is separated from the gas phase. The condensate water 13 can be utilized internally in the rWGS/reforming integrated unit for process steam generation, recycled to the electrolysers (3) or treated in a waste water treatment plant. It should be noted that the condensate can contain some contaminants for the electrolyzer and might require additional pre-treatment steps to remove these impurities. The purification steps are not shown.

The cooled syngas 11 contains mostly CO, H2, unconverted CO2 and CH4 and some nitrogen and/or other inerts and is saturated with water. This syngas stream is further treated in a purification and conditioning unit 12. This conditioning can include CO2 capture, hydrogen removal, methane removal or other purification process to clean the syngas for the downstream synthesis unit. As a result of the thermodynamic equilibrium of the WGS reaction, there is still a signification portion of CO2 in this stream. If this not allowable for the downstream unit (for example if CO2 is a poison or an inert), the CO2 can be captured in a CO2 capture unit, this could for example be a membrane, Vacuum Swing Adsorption (VSA), Pressure Swing Adsorption (PSA), cryogenic, amine adsorption process or any conventional CO2 capture process for extracting CO2 from syngas. The CO2 from the capture unit 13 is recycled back to the rWGS reactor as a feedstock, for this reason hydrogen in the CO2 stream is also allowed. In case there is an excess of hydrogen in the conditioned syngas to the synthesis unit, hydrogen could be extracted by for example a membrane unit and recycled 14 to the rWGS unit as well. In case of a shortage of hydrogen in the syngas additional hydrogen make-up 15 can be added to the syngas.

The purified and conditioned syngas 16 is fed to the downstream synthesis unit 17. In the synthesis unit the syngas reacts over a catalyst to produce synthetic hydrocarbons. In a preferred embodiment the synthesis unit is a Fischer Tropsch (FT) unit, producing synthetic fuels including but not limited to LPG, gasoline, diesel, waxes and in particular preferable Jet Fuel. Alternatively, the synthesis unit could also consist of methanol, Methanol to gasoline/jet fuel, higher alcohol, syngas to olefins, syngas to ethylene oxide or combined synthesis. In case of low single pass conversion an internal recycle of unconverted syngas is included in the synthesis unit. Any excess energy, for example from the exothermic FT reaction that cannot be utilized internally is recovered as steam. In FT typically medium pressure steam is produced. This steam can be utilized in the upstream and downstream reboilers to minimize exergy losses in the system.

The synthesis product gas 18 contains a wide range of components and further treated in a separation and conditioning unit 19, which includes cooling down the gas, condensation of water, further reaction (for example hydrogenation, hydrocracking, isomerization and aromization) and separation/ purification of the final product. The reactor units as part of the conditioning might require additional feed gasses, such as hydrogen for hydrogenation and hydrocracking). These units are typically included to increase the product yield and/or quality. The water condensed in conditioning unit is separated into a condensate stream (26). This stream can be utilized internally in the rWGS/reforming integrated unit for process steam generation, recycled to the electrolysers or treated in a waste water treatment plant. It should be noted that the condensate can contain some contaminants for the electrolyzer and might require additional pre-treatment steps to remove these impurities. The purification steps are not shown. The light offgasses of the process, including some unconverted syngas, light hydrocarbons (typically C1-C5), CO2 and other impurities are separated from the products streams. The light offgas stream 22 is recycled back to the reforming section 7. The product stream 21 comprises the desired products of the synthesis unit. For the example of FT synthesis, the desired products include LPG, gasoline, diesel, jet fuel and waxes.
The light offgas 22 can optionally be further purified before being fed to the reforming section 7 in a purification section 23. This section typically can contain a CO2 capture unit, comprising a membrane, Vacuum Swing Adsorption (VSA), Pressure Swing Adsorption (PSA), cryogenic, amine adsorption process or any conventional CO2 capture process for extracting CO2.ln case of CO2 capture, the CO2 stream 25 is recycled on combined with the other CO2 stream as feed for the rWGS option. This can be of particular benefit for streams with a high CO2 partial pressure (>10vol% of CO2) as this reduced the risk of carbon formation in reforming section as well as the steam requirement in the reforming section. In case the hydrocarbons offgas is very rich in hydrogen it is possible to first apply a hydrogen separation step (for example a membrane) before feeding the hydrocarbon offgas to the reforming section.

In the reforming section, offgas stream 24 is preheated and mixed with steam generated in the integrated reforming and rWGS sections, before being fed to a reforming reaction unit. The reforming reaction unit could be either one or a combination of the following units, prereformer, steam methane reformer (SMR), autothermal reformer (ATR) or partial oxidation (POX). In the reforming reaction unit, the hydrocarbons in the offgas are converted into a mixture of CO, H2, CO2, H2O and some residual methane. The outlet temperature of the reformer section is preferably more than 700°C, more preferably more than 900°C as this maximizes the hydrocarbon conversion as well as a high driving force for the rWGS reaction. The hot effluent from the reforming section 8, the reformer effluent, is directly mixed with the shift effluent inside the rWGS reactor as described above.

In case the reforming reaction section contains a SMR the heat required for the reaction can be provided by either a fired heater, oxycombustion heater and/or and electrical heater. In case of a fired/oxycombustion heater, at least part of the oxygen from the electrolysers can be utilized in the combustion system The fuel for this system could be the light offgas, but is preferably hydrogen. In case H2 fuel is used, the carbon loop of the overall process is completely closed. In case the reforming section contains an ATR or POX at least part of the oxygen from electrolysers 20 can be utilized in the reactor. This avoids the requirement for an air separation unit.

The integrated system is designed such that the offgas reforming provides sufficient heat to drive the rWGS reaction and no additional system is required. In case the light hydrocarbons in the offgas are insufficient to provide all the duty for the rWGS section, it is possible to partially electrify the rWGS or to utilize an additional hydrocarbon import stream (not shown). This import hydrocarbon is preferably from biological source, for example an offgas from an adjacent biofuel plant or synthetic natural gas (for example produced by methanation of CO2 with hydrogen).

An essential part of the invention is that the rWGS heat exchanger reactor is integrated with the reforming section. Thereby minimizing the total (external) energy demand of the system. At the same time is also enables closing of both the hydrogen and carbon balance of the complete process. As part of the required syngas is produced from the offgasses of the downstream synthesis unit, subsequently the amount of syngas produced the rWGS reaction can be reduced and thus requiring less carbon dioxide and less hydrogen import for the same quantity of products. The reduction in CO2 import, means that more product can be produced from the same CO2 source. This brings especially benefits in energy consumption in case the CO2 is captured using DAC, which significant energy input to capture the carbondioxide.

Similarly the reduction in import hydrogen reduces the size and energy required for the electrolysers. The conversion of water into hydrogen (and oxygen) is electricity intensive and requires and an energy input around 8 times as high as the hydrogen produced from the offgasses. The overall energy intensity thus decreases. An additional benefit is that with the reduction of hydrogen demand it also reduces the import water required to the plant and therewith the size of the water purification systems as well as the scarce water resources. Additional benefits on the water reduction can be achieved by recycling condensate from the syngas and the synthesis product to the electrolysers. The utilization of the offgas for producing part of the syngas also allows the system to be free of direct CO2 emissions, while at the same time also reducing the scope 2 carbon emissions (mostly in power generation) and in case of DAC result in a negative CO2 emission.
The table 1 shows an example of the present invention with a not integrated rWGS scheme in combination with Fischer Tropsch synthesis. The Fischer Tropsch synthesis is based on a conventional FT synthesis which targets higher hydrocarbons (mostly waxes) and subsequent hydrocracking to produce mainly synthetic gasoline and jet fuel. For this the Fischer Tropsch Unit is not compared with a traditional process include CtL and GTL as both of these processes require fossil fuels as input. The example is a based on a 1000 barrel per day product flowrate
1) Basecase: this includes an electrolyzer for producing hydrogen combined with import CO2 fed to a rWGS reactor and subsequently a FT synthesis and separation unit. The offgas is utilized for a combined gas turbine cycle to generate power.
2) The invention including an SMR based reformer unit integrated with the parallel rWGS heat exchanger reactor
3) A second embodiment of this invention including a prereformer and ATR based reforming section integrated with the parallel rWGS heat exchanger reactor. The ATR utilizes part of the oxygen from the electrolysers.

**Table 1**

| | Base Case | Invention Case 1 | Invention Case 2 |
|---|---|---|---|
| CO2 import [KTPA] | 241 | 122 | 122 |
| Hydrogen from electrolyzer [kNm3/h] | 38.8 | 28.0 | 27.2 |
| Power consumption [GWh per year]1) | 1647 | 1189 | 1153 |
| CO2 Emission [KTPA]2) | 152 | 23.8 | 23.1 |
| Water import [KTPA]3) | 103 | 112 | 77 |

| | | | |
|---|---|---|---|
| KTPA = Kilo Tonnes Per Annum 1) Power consumption is expected to be around 90% dominated by the electrolyzer power consumption 2) Based on scope 1 and 2 emissions with an electricity carbon intensity of 20gCO2/kWh 3) Including condensate recycling | | | |

The basecase with does not close the complete carbon loop as the light hydrocarbons are not recycled to the rWGS process. The total carbon import required is therefore around 50% lower for the current inventions. In other words for the same CO2 feedstock the current invention would allow approximately two times the product flowrate. The hydrocrabons in the light ofggasses are utilized in a combined cycle gas turbine to generate power and the CO2 generated is emitted through the flue gas. It should be noted that it is possible to capture a significant portion of the CO2 from the flue gas and subsequently recycled back to the rWGS feed lowering CO2 import to close to the current invention at the cost of additional energy input for the carbon capture unit. As the flue gas exhaust also is a direct emission of CO2 to the atmosphere, the CO2 emission would also decrease by around 110KTPA.

The main advantage of the current invention over the basecase is the reduction of the required hydrogen. As the recycled light hydrocarbons also contain hydrogen, the reforming section converts the hydrocarbons into hydrogen and CO and subsequently up to around 25% reduction of hydrogen from electrolysers is required in case 1. In case of a reforming section containing autothermal reformer this can be reduced even further with around 30% compared to the basecase. This is feasible due to the utilization of the oxygen form the electrolysers and the higher outlet temperature form the ATR. This also allows a lower methane slip and higher conversion rate (both in reformer and rWGS section) and subsequently lower hydrogen requirement.

As typically the majority of the electricity consumption is related to the hydrogen production in the electrolysers the same is reflected in the total power consumption. Current electrolysers technologies have a specific power consumption of around 50-60kW/kg H2 which results in 70-95% of the total power consumption depending on the detailed lay-out of the system and the source of the CO2. As such the savings in hydrogen from the integrated system saves a significant portion of power. Furthermore as the rWGS is fully integrated with the reforming section no external heat input (in the form of power or fired heater) is required for the rWGS reactor. Thereby saving an additional 3-5% of electricity and thus of total energy input and global CO2 footprint.

The particular benefit of the proposed system compared to other previous publications is that on top of the reduction in power and energy footprint the additional benefit is on the operational benefits of the rWGS integration. The parallel reaction of the reforming section and rWGS maximizes the conversion to CO and minimizes the H2 import as the equilibrium reaction is at more favorable conditions (for methane conversion and for CO2 conversion) then when the two stream are combined before the reforming or rWGS catalyst. The additional benefit of this system is that the mixing of the rWGS effluent with the reformer effluent inside the rWGS heat exchanger reactor reduces the risk of carburization and corrosion compared to a separate rWGS effluent, especially in the temperature range of 600-800°C.

It should be noted that, in particular cases, the C3-C5 hydrocarbons are recovered as LPG product and therefore the light hydrocarbon recycle is reduced. The benefits of the present invention remain though it should be noted that the CO2 import will come closer together.

To summarize the main advantages of the present invention:
- The main advantage over the basecase is the reduction of the required hydrogen. As the recycled light hydrocarbons also contain hydrogen, the reforming section converts the hydrocarbons into hydrogen and CO and subsequently up to around 25% reduction of hydrogen from electrolysers is required in case 1. In case of a reforming section containing autothermal reformer this can be reduced even further with around 30% compared to the basecase. This is feasible due to the utilization of the oxygen form the electrolysers and the higher outlet temperature form the ATR. This also allows a lower methane slip and higher conversion rate (both in reformer and rWGS section) and subsequently lower hydrogen requirement.
- As typically the majority of the electricity consumption is related to the hydrogen production in the electrolysers the same is reflected in the total power consumption. Current electrolysers technologies have a specific power consumption of ~50-60kW/kg H2 which results in 70-95% of the total power consumption depending on the detailed lay-out of the system and the source of the CO2. As such the savings in hydrogen from the integrated system saves a significant portion of power. Furthermore as the rWGS is fully integrated with the reforming section no additional external heat input (in the form of power or fired heater) is required for the rWGS reactor. Thereby saving an additional 3-5% of electricity and thus of total energy input and global CO2 footprint.
- The particular benefit of the proposed system compared to other previous publications is that on top of the reduction in power and energy footprint the additional benefit is on the operational benefits of the rWGS integration. The parallel reaction of the reforming section and rWGS maximizes the conversion to CO and minimizes the H2 import as the equilibrium reaction is at more favorable conditions (for methane conversion and for CO2 conversion) then when the two stream are combined before the reforming or rWGS catalyst. The additional benefit of this system is that the mixing of the rWGS effluent with the reformer effluent inside the rWGS heat exchanger reactor reduces the risk of carburization and corrosion compared to a separate rWGS effluent, especially in the temperature range of 600-800°C.
- Compared to processes that do start with fossil fuel sources (for example gas, coal) there is a carbon neutral or even negative CO2 footprint, especially if DAC is applied.
- In case the recycle hydrocarbon offgas is not sufficient to provide all the heat, it is possible to co-feed a part of the CO2 to the reforming feed. Requiring a slight increase of S/C ratio in the reformer. Alternatively other hydrocabrons feedstocks can be utilized as a feed to the unit.
- An additional advantage of the current invention is that the reforming section can handle a very wide range of feedstocks from light hydrogen rich off gases through LPG and up to light naphtha, As such it also enables the plant to utilizes any undesired product as a feedstock for the reforming section and keep the carbon loop closed.

## Claims

1. System for producing synthetic fuels comprising:
- A reforming reactor (7) configured to react a light hydrocarbon feed (24) together with an oxidant gas stream (20) thereby producing a reformer effluent comprising a synthesis gas stream, and
- A reverse water gas shift reactor (6) configured to receive a CO2 rich stream, a H2 rich stream and the reformer effluent and to produce a synthesis gas with a reduced H2/CO ratio,
- A synthesis unit (17) configured to receive the synthesis gas with a reduced H2/CO ratio and to produce a feed comprising synthetic fuels (18),
With the reverse water gas shift reactor (17) comprising a vessel comprising:
• at least one catalyst zone, preferably a catalyst tube, configured to receive the H2 rich stream and the CO2 rich stream and to produce a shift effluent enriched in CO and H2O,
• a heat transfer zone configured to receive the reformer effluent and to transfer the heat of the reformer effluent to the catalyst zone,
and
means configured to mix the shift effluent with the reformer effluent and to produce the synthesis gas with the reduced H2/CO ratio.

2. System according to claim 1, wherein the synthesis unit (17) is any of the following units or a combination of any of these: a methanol synthesis unit, a methanol synthesis unit combined with a methanol to gasoline or methanol to jet fuel unit, a syngas to olefines synthesis unit, a syngas to ethyleneoxide unit and a Fischer Tropsch (FT) unit.

3. System according to claim 1 or claim 2, wherein it comprises at least a separation unit (19) configured to receive the feed comprising synthetic fuels (18) coming from the synthesis unit (17) and to produce synthetic fuels (21), water (26) and a feed comprising light hydrocarbons (22).

4. System according to claim 3, wherein it comprises:
- a first CO2 capture unit (23) configured to receive the feed comprising light hydrocarbons and to produce CO2 stream (25) and light hydrocarbons (24); and
- Preferably the reforming reactor (7) is configured to receive the light hydrocarbons coming from the CO2 capture unit (23), and/or
- Preferably the reverse water gas shift reactor (6) is configured to receive the CO2 stream (25) coming from the CO2 capture unit (23).

5. System according to any of the claims 1 to 4, wherein:
- it comprises an electrolyzer unit (3) configured to receive water (1) and to produce a H2 stream (4) and a O2 stream (20),
- the reverse water gas shift reactor (6) is configured to receive the H2 stream (4) coming from the electrolyzer unit (3), and
- preferably the reforming reactor (7) is configured to receive the O2 stream coming from the electrolyzer unit (3) as oxidant gas stream (20).

6. System according to any of the claims 1 to 5, wherein the end of the catalyst zone opens in the heat transfer zone and means to mix configured to mix the shift effluent with the reformer effluent are located in the heat transfer zone of the vessel.

7. System according to any of the claims 1 to 5, wherein the end of the catalyst zone opens in a zone comprised in the vessel and fluidically isolated from the heat transfer zone and means to mix the shift effluent with the reformer effluent are located at the reverse water gas shift reactor outlet.

8. System according to any of the claims 1 to 7, wherein:
- The system comprises a second CO2 capture unit (12) configured to receive the synthesis gas with the reduced H2/CO ratio and to produce CO2 stream (13) and a synthesis gas (16) depleted in CO2 and with the reduced H2/CO ratio, and
- Preferably the catalyst zone is configured to receive a CO2 stream coming at least in part from the CO2 capture unit (12).

9. Process for producing synthetic fuels, said process implementing a system according to any of the claims 1 to 8 and comprises:
a) Reforming step (7) of a hydrocarbon feed (24) with an oxidant gas stream (20) to produce a reformer effluent comprising a synthesis gas stream,
b) Heating step of the catalyst zone of the of the reverse water gas shift reactor by exchange with the reformer effluent,
c) Reverse water gas shift reaction step (6) of a CO2 rich stream and a H2 rich stream (5) in the catalyst zone of the reverse water gas shift reactor to produce a shift effluent enriched in CO and H2O,
d) Mixing step of the shift effluent with the reformer effluent to produce a synthesis gas with the reduced H2/CO ratio (9),
e) Synthesis step (17) of a feed comprising synthetic fuels (18) from the synthesis gas with a reduced H2/CO ratio (9).

10. Process according to claim 9, wherein the synthesis step is a synthesis step according to the Fischer Tropsch process.

11. Process according to claim 9 or claim 10, wherein it comprises a separation step of the feed comprising synthetic fuels (18) to produce synthetic fuels (21), water (26) and a feed (22) comprising light hydrocarbons.

12. Process according to any of the claims 9 to 11, wherein it comprises:
- a first step of capture of CO2 comprised in the feed (22) comprising light hydrocarbons to produce CO2 stream (25) and light hydrocarbons (24),
- Preferably a recycling step of light hydrocarbons (24) in the reforming reactor (7), and
- Preferably a recycling step of the CO2 stream (25) in the reverse water gas shift reactor (6).

13. Process according to any of claims 9 to 11, wherein:
- it comprises an electrolysis step of water (1) to produce H2 stream (4) and a O2 stream (20),
- Preferably the O2 stream produced at the electrolysis step is used as oxidant gas stream (20) in the reforming step (7), and
- the H2 stream produced at the electrolysis step is introduced in the reverse water gas shift reactor (6).

14. Process according to any of the claims 9 to 13, wherein it comprises a cooling step of the synthesis gas (9) coming from step d).

15. Process according to any of the claims 9 to 14, wherein it comprises:
- a second step of capture of CO2 comprised in the synthesis gas with the reduced H2/CO ratio (9) to produce CO2 stream (13) and a synthesis gas (16) depleted in CO2 and with the reduced H2/CO ratio, and
- a recycling step of the CO2 stream (13) in the reverse water gas shift reactor (6).
